# EUROPEAN PATENT APPLICATION

(11) **EP 4 265 630 A1**
(43) Date of publication of application: **25.10.2023**
(21) Application number: 21907124.8
(22) Date of filing: 16.12.2021
(51) Int. Cl.: C07K 7/08, A61K 47/64, A61K 47/56, A61Q 19/00, A61K 8/64, A61K 47/54, A61P 17/00

(54) **ANTI-INFLAMMATORY PEPTIDE FOR PREVENTING OR TREATING ATOPIC DERMATITIS**

(30) Priority: 16.12.2020 KR 20200176501; 15.12.2021 KR 20210179908
(71) Applicant: Ajou University Industry-Academic Cooperation Foundation, Suwon-si, Gyeonggi-do 16499 (KR)
(72) Inventor: SOHN, Seong Hyang, Suwon-si Gyeonggi-do 16699 (KR)
(74) Representative: Roos, Peter
(86) International application number: PCT/KR2021/019190
(87) International publication number: WO 2022/131820

(57) **Abstract**

The present invention relates to an anti-inflammatory peptide for preventing or treating atopic dermatitis, and specifically, the present invention provides: a peptide or fragment thereof consisting of an amino acid sequence selected from the group consisting of SEQ ID NO: 1 to SEQ ID NO: 17, or a conjugate thereof; and a pharmaceutical composition and a cosmetic composition for preventing or treating atopic dermatitis comprising the peptide or fragment thereof, etc. as an active ingredient. The peptide or fragment thereof, or a conjugate thereof, has excellent anti-inflammatory activity, and thus is safe from the side effects of existing therapeutic agents for atopic dermatitis, and can effectively prevent, ameliorate, or treat atopic dermatitis.

## Description

### Technical Field

The present disclosure relates to an anti-inflammatory peptide for preventing or treating of atopic dermatitis.

### Background Art

Atopic dermatitis is a chronic, intractable skin inflammatory disease that is common in children with symptoms persistable into adulthood. It is usually accompanied by pruritus (itching), dry skin, and specific eczema, and especially, repetitive scratching actions due to severe itching may cause damage to the skin barrier, worsen inflammation, and aggravate itching, entering a continuous vicious cycle. Dry skin also causes and exacerbates itching.

Estimated main causes of atopic dermatitis are environmental factors, genetic predisposition, immunological abnormalities, and abnormalities in the skin protective barrier, but the cause of occurrence is not yet clearly known, posing a difficulty in finding and treating the root cause of atopic dermatitis. Therefore, rather than aiming for complete curing of atopic dermatitis, it is general to avoid triggers and control symptoms of atopic dermatitis through appropriate treatment.

Currently, steroids and calcineurin inhibitors are the most applied as therapeutic agents for atopic dermatitis. In addition, antihistamines that suppress itching and antibiotics are used to prevent secondary bacterial infections. Although steroids (adrenocorticosteroids) exhibit outstanding effects owing to anti-inflammatory and immunosuppressive actions, long-term application may cause side effects such as skin weakening, systemic hormone symptoms, and addictiveness. Calcineurin inhibitors may be applied onto thin and weak skin, but the use thereof is limited due to carcinogenesis in infants and children. In addition, antihistamines may be used as a temporary measure to relieve itchy symptoms by preventing histamine from being released from mast cells, but side effects may appear such as sleeplessness, anxiety, and a loss of appetite in the long-term application.

Recently, Elidel Cream 1% (ingredient: pimecrolimus), a nonsteroidal atopic dermatitis immunotherapy approved by the U.S. FDA in December 2001, has been prescribed as a therapeutic agent for atopic dermatitis in the U.S. and around the world, and PDE-4 inhibitors have been released. However, a problem remains concerning the recurrence rate that reaches 45% after 4 weeks of treatment discontinuation with PDE-4 inhibitor.

Therefore, there is a need for a safe, effective therapeutic agent for atopic dermatitis without these side effects.

### Disclosure of the Invention

### Technical Goals

An object of the present disclosure is to provide a novel peptide having an anti-inflammatory effect.

Another object of the present disclosure is to provide a composition for preventing or treating atopic dermatitis, including the peptide as an active ingredient.

### Technical Solutions

In order to achieve the above object, the present disclosure provides a peptide or fragment thereof having an amino acid sequence selected from the group consisting of SEQ ID NO: 1 to SEQ ID NO: 17.

The present disclosure provides a conjugate in which the peptide or fragment thereof is conjugated with a biocompatible polymer or fatty acid.

The present disclosure provides a pharmaceutical composition for preventing or treating atopic dermatitis, including, as an active ingredient, one or more selected from the group consisting of the peptide or fragment thereof, and the conjugate.

In addition, the present disclosure provides a cosmetic composition for preventing or ameliorating atopic dermatitis, including, as an active ingredient, one or more selected from the group consisting of the peptide or fragment thereof, and the conjugate.

### Advantageous Effects

A novel peptide or fragment thereof, or the conjugate thereof according to the present disclosure, has an outstanding anti-inflammatory activity and thus may be utilized as a pharmaceutical composition or cosmetic composition for preventing, treating, or ameliorating atopic dermatitis.

By utilizing the composition, it is possible to prevent, treat, or ameliorate atopic dermatitis safely and more effectively from side effects of conventional therapeutic agents for atopy.

### Brief Description of Drawings

FIG. 1 shows experimental results of atopic dermatitis-induced mouse models.
FIG. 2 shows results of analyzing anti-inflammatory activity of peptides using splenocytes.
FIG. 3 shows results of analyzing anti-inflammatory activity of peptides using peripheral blood leukocytes (PBLs).
FIG. 4 shows a result of analyzing an IL-4 expression level after treating atopic dermatitis-induced mice with peptides.
FIG. 5 shows results of analyzing skin tissue penetrability of a peptide.
FIG. 6 shows results of analyzing, via an in vivo experiment, the frequency of peritoneal macrophage expressing peptides in cells, obtained by injecting fluorescently labeled peptides into the abdominal cavity and then isolating peritoneal macrophages by time.
FIG. 7 shows results of analyzing, via an in vivo experiment, the intracellular expression and frequency after treating peptides while culturing isolated lymph node cells of mice.
FIG. 8 shows results of analyzing, via an in vivo experiment, the intracellular expression and frequency after treating peptides while culturing isolated peritoneal macrophages of mice.
FIG. 9 shows results of analyzing effects of peptides in the spleen after treating mice having atopic dermatitis with peptides in a form of external preparation (A900~A916).
FIG. 10 shows results of FACS analysis of T cells associated with a cause of atopic dermatitis symptoms after treating peptides or variants thereof while culturing isolated peripheral blood mononuclear cells (PBMCs) in mice having atopic dermatitis.
FIG. 11 shows results of analyzing an expression level of cytokines associated with a cause of atopic dermatitis symptoms after treating peptides or variants thereof while culturing isolated splenocytes in mice having atopic dermatitis.
FIG. 12 shows results of analyzing an expression level of cytokines associated with a cause of atopic dermatitis symptoms after treating peptides or variants thereof while culturing isolated lymph node cells in mice having atopic dermatitis.
FIG. 13 shows results of analyzing, via an in vivo experiment, effects of peptides through the frequency of cytokine-expressing cells, obtained by treating a peptide A900 or variant thereof in a form of external preparation in mice having atopic dermatitis and then isolating peripheral blood mononuclear cells (PBMCs).
FIG. 14 shows results of analyzing, via an in vivo experiment, effects of peptides through the frequency of cytokine-expressing cells among CD4 T cells, obtained by treating a peptide A900 or variant thereof in a form of external preparation in mice having atopic dermatitis and then isolating peripheral blood mononuclear cells (PBMCs).

### Best Mode for Carrying Out the Invention

Hereinafter, the present disclosure will be described in detail.

The present inventor completed the present disclosure by preparing a novel peptide to identify an anti-inflammatory activity thereof and determine a therapeutic effect in atopic dermatitis-induced mouse models.

The present disclosure provides a peptide or fragment thereof having an amino acid sequence selected from the group consisting of SEQ ID NO: 1 to SEQ ID NO: 17.

The peptide or fragment thereof has an immunoregulatory function or anti-inflammatory activity.

In addition, the peptide or fragment thereof penetrates skin tissues and passes through cell membranes.

In addition, the peptide or fragment thereof inhibits the frequency of CD4 T cells to suppress cytokines, thereby having an activity in atopic dermatitis.

The cytokine may be one or more selected from the group consisting of IL-4, IL-13, IL-17, IL-21, and IL-31, but is not limited thereto.

The letters used in the amino acids refer to the following amino acids according to the standard abbreviation regulations in the field of biochemistry.

A(Ala): alanine; C(Cys): cysteine; D(Asp): aspartic acid; E (Glu): glutamic acid; F(Phe): phenylalanine; G(Gly): glycine; H(His): histidine; I(IIe): isoleucine; K(Lys): lysine; L(Leu): leucine; M(Met): methionine; N(Asn): asparagine; O(Ply): pyrrolysine; P(Pro): proline; Q(Gln): glutamine; R(Arg): arginine; S(Ser): serine; T(Thr): threonine; U(Sec): selenocysteine, V(Val): valine; W (Trp): tryptophan; Y(Tyr): tyrosine.

As used herein, the term "peptide or fragment thereof" refers to a polymer consisting of two or more amino acids linked by an amide bond (or peptide bond).

The peptide or fragment thereof according to the present disclosure may have an immunoregulatory function or anti-inflammatory activity.

According to one experimental example of the present disclosure, it was found that the increased frequency of IL-4R alpha, CCR3, and CTLA-4 positive cells in mice having atopic dermatitis all decreased after treatment of a peptide A900, and cytokines such as IL-13, IL-4, TNF alpha, and IL-17 were also reduced.

The peptide or fragment thereof consisting of an amino acid sequence selected from the group consisting of SEQ ID NO: 1 to SEQ ID NO: 17 according to the present disclosure may be derived from natural sources and may be chemically synthesized using chemical synthesis methods or genetic engineering methods known in the art, or may be obtained using gene recombination techniques, but is not limited thereto.

Typical methods for the above known chemical synthesis (Creighton, Proteins; Structures and Molecular Principles, W. H. Freeman and Co., NY, 1983) include liquid or solid phase synthesis, fragment condensation, F-MOC, or T-BOC chemical methods (Chemical Approaches to the Synthesis of Peptides and Proteins, Williams et al., Eds., CRC Press, Boca Raton Florida, 1997; A Practical Approach, Athert on & Sheppard, Eds., IRL Press, Oxford, England, 1989), but are not limited thereto.

The genetic engineering method may enable construction of a DNA sequence that encodes the peptide or fragment thereof according to conventional methods. The DNA sequence may be constructed by PCR amplification using appropriate primers. Alternatively, DNA sequences may be synthesized via standard methods known in the art, such as automated DNA synthesizers (sold by Biosearch or Applied Biosystems).

The synthesized DNA sequence is operably linked thereto and inserted into a vector including one or more expression control sequences that regulate expression of DNA sequences, and transforms host cells using the recombinant expression vector formed therefrom to produce a transformant. The produced transformant is cultured in a medium and conditions appropriate to express the DNA sequence to harvest a substantially pure peptide encoded by the DNA sequence from a culture. The harvesting may be performed using a method known in the art (such as chromatography).

The peptide according to the present disclosure may include not only a peptide having a natural type of amino acid sequences but also an amino acid sequence variant thereof.

The term "amino acid sequence variant" as used herein refers to a peptide having a different sequence of one or more amino acid residues in the amino acid sequence according to the present disclosure by deletion, insertion, non-conservative or conservative substitution, substitution of amino acid analogs, or combinations thereof, but the activity of a molecule is not changed as a whole by the above mutation.

In addition, the peptide according to the present disclosure may be modified by phosphorylation, sulfation, acrylation, glycosylation, methylation, and farnesylation.

In addition, the present disclosure provides a conjugate in which a peptide or fragment thereof consisting of an amino acid sequence selected from the group consisting of SEQ ID NO: 1 to SEQ ID NO: 17 is conjugated with a biocompatible polymer or fatty acid.

The biocompatible polymer may be one or more selected from the group consisting of pullulan, chondroitin sulfate, hyauronic acid (HA), glycol chitosan, starch, chitosan, dextran, pectin, curdlan, poly-L-lysine, poly-aspartic acid (PAA), polylactic acid (PLA), polyglycolide (PGA), poly(ε-caprolactone) (PCL), poly(caprolactone-lactide) random copolymer (PCLA), poly(caprolactone-glycolide) random copolymer (PCGA), poly(lactide-glycolide) random copolymer (PLGA), polyethylene glycol (PEG), pluronic F-68, and pluronic F-127, but is not limited thereto.

The fatty acid may be one or more selected from the group consisting of hexanoic acid, caprylic acid (C8), capric acid (C10), lauric acid (C12), myristic acid (C14), palmitic acid (C16) stearic acid (C18), and cholesterol, but is not limited thereto.

By conjugating the peptide or fragment thereof with the biocompatible polymer or fatty acid, a specific purpose such as increasing the stability of the peptide may be performed. For example, the peptide or fragment thereof may be conjugated (PEGylation) with polyethylene glycol (PEG), a biocompatible polymer, to make the peptide or protein water soluble or improve the half-life.

The present disclosure provides a pharmaceutical composition for preventing or treating atopic dermatitis, including, as an active ingredient, one or more selected from the group consisting of a peptide or fragment thereof consisting of an amino acid sequence selected from the group consisting of SEQ ID NO: 1 to SEQ ID NO: 17, and a conjugate in which the peptide or fragment thereof is conjugated with a biocompatible polymer or fatty acid.

The composition is selected from the group consisting of oral preparations, external preparations, suppositories, and injections, but is not limited thereto.

The peptide or fragment thereof, or the conjugate has an outstanding anti-inflammatory activity and thus may be used as a pharmaceutical composition for preventing or treating atopic dermatitis.

The peptide or fragment thereof, or the conjugate may be included in an amount of 0.001 to 50 parts by weight with respect to 100 parts by weight of the total composition, but is not limited thereto.

The pharmaceutical composition according to the present disclosure may be prepared according to conventional methods in the pharmaceutical field.

The pharmaceutical composition may be combined with an appropriate pharmaceutically acceptable carrier according to a formulation and may be prepared, as needed, by further including excipients, diluents, dispersants, emulsifiers, buffers, stabilizers, binders, disintegrants, and solvents.

As used herein, the term "pharmaceutically acceptable" refers to a state non-toxic to cells or humans exposed to the pharmaceutical composition, and the appropriate carrier may not interfere with the activity and properties of the peptide or fragment thereof, or the conjugate according to the present disclosure, which may be selected differently according to the dosage form and formulation.

The pharmaceutical composition according to the present disclosure may be applied in any formulation, and more specifically, it may be used by being formulated in parenteral formulations of oral formulations, external preparations, suppositories, and sterile injection solutions according to conventional methods.

A solid formulation of the oral formulation may be prepared in the form of tablets, pills, acids, granules, and capsules by mixing with at least one or more excipients such as starch, calcium carbonate, sucrose, lactose, sorbitol, mannitol, cellulose, and gelatin and may include lubricants such as magnesium stearate and talc in addition to simple excipients. In addition, in the case of the capsule formulation, liquid carriers such as fatty oil may be further included in addition to the above-mentioned substances.

Liquid formulations of the oral formulations include suspensions, solutions, emulsions, and syrups, and various excipients such as wetting agents, sweeteners, fragrances, and preservatives may be included in addition to commonly used simple diluents such as water and liquid paraffin.

The parenteral formulation may include sterilized aqueous solutions, non-aqueous solvents, suspensions, emulsions, lyophilized preparations, and suppositories. Propylene glycol, polyethylene glycol, vegetable oils such as olive oil, and injectable esters such as ethyl oleate may be used as non-aqueous solvents and suspensions. Witepsol, macrogol, Tween 61, cacao butter, laurin fat, and glycerogelatin may be used as a base of the suppositories. Without limitation, all suitable preparations known in the art may be used.

In addition, in the pharmaceutical composition according to the present disclosure, calcium or vitamin D3 may be added further to enhance a therapeutic efficacy.

The pharmaceutical composition according to the present disclosure may be administered in a pharmaceutically effective amount.

As used herein, the term "pharmaceutically effective amount" refers to an amount sufficient to treat a disease at a reasonable benefit/risk ratio applicable to medical treatment without causing side effects.

The effective dose level of the pharmaceutical composition may be determined differently, depending on factors including the purpose of use, the age, sex, weight, and health status of a patient, the type of disease, severity, the activity of a drug, the sensitivity to the drug, the method of administration, duration of administration, administration routes and excretion rate, treatment periods, and drugs used in combination or simultaneously, and other well-known factors in the medical field. For example, although not constant, it may be administered generally in a dose of 0.001 to 100 mg/kg, preferably 0.01 to 10 mg/kg once to several times a day. The dosage does not limit the scope of the present disclosure in any way.

The pharmaceutical composition according to the present disclosure may be administered to any animal in which atopic dermatitis may occur, and the animal may include, for example, not only humans and primates, but also livestock such as cattle, pigs, horses, and dogs.

The pharmaceutical composition according to the present disclosure may be administered by a suitable route of administration according to the formulation form and may be administered through various routes such as oral or parenteral forms, as long as it may reach the desired tissue. The method of administration may be conducted via conventional methods without particular limitation, for example, oral, rectal or intravenous, intramuscular, subcutaneous, bronchial suction, intrauterine dural, or intracere-broventricular injections.

The pharmaceutical composition according to the present disclosure may be solely used for prevention or treatment of atopic dermatitis and may be used in combination with surgery or other drug treatment.

The present disclosure provides a cosmetic composition for preventing or ameliorating atopic dermatitis, including, as an active ingredient, one or more selected from the group consisting of a peptide or fragment thereof consisting of an amino acid sequence selected from the group consisting of SEQ ID NO: 1 to SEQ ID NO: 17, and a conjugate in which the peptide or fragment thereof is conjugated with a biocompatible polymer or fatty acid.

The peptide or fragment thereof, or the conjugate has the outstanding anti-inflammatory activity and may be used as a cosmetic composition for preventing or ameliorating atopic dermatitis.

The peptide or fragment thereof, or the conjugate may be included in an amount of 0.001 to 50 parts by weight with respect to 100 parts by weight of the total composition, but is not limited thereto.

The cosmetic composition has a formulation of softening toner, nourishing toner, nourishing cream, gel, essence, soap, ointment, balm, or pack.

The cosmetic composition according to the present disclosure may further include one or more of ingredients listed in the cosmetic ingredient list registered in the Ministry of Food and Drug Safety and International Cosmetic Ingredient Dictionary (ICID). For example, the cosmetic composition may further include one or more of adjuvants commonly used in the cosmetic field such as organic solvents, solubilizers, thickeners, gelling agents, softeners, antioxidants, suspensions, stabilizers, aromatics, surfactants, emulsifiers, fillers, metal ion blockers, preservatives, vitamins, blockers, wetting agents, dyes and pigments, hydrophilic or lipophilic active agents, or any other ingredient commonly used in cosmetics.

The vitamin may include water-soluble or oil-soluble vitamins, wherein the water-soluble vitamin may be combined in cosmetics as a water-soluble vitamin, preferably vitamin B1, vitamin B2, vitamin B6, pyridoxine, pyridoxine hydrochloride, vitamin B12, pantothenic acid, nicotinic acid, nicotinamide, folic acid, vitamin C, and vitamin H. In addition, salts thereof (thiamine hydrochloride, sodium ascorbate) or derivatives (ascorbic acid-2-sodium phosphate, ascorbic acid-2-magnesium phosphate) may also be included, and the water-soluble vitamins may be obtained by conventional methods such as microbial conversion methods, purification methods from cultures of microorganisms, enzyme methods, or chemical synthesis methods.

The cosmetic composition according to the present disclosure may be prepared in any formulation commonly prepared in the art to which the present disclosure pertains. For example, it may be formulated into toner, emulsion, lotion, cream, paste, gel, solution, suspension, oil, wax, pack, powder, foundation, spray, and surfactant-containing cleansing, but is not limited thereto.

More specifically, it may be prepared in formulations of softening toner, nourishing toner, astringent toner, nourishing cream, massage cream, milk lotion, powder, essence, eye cream, sun lotion, sun cream, makeup primer, makeup base, BB cream, powder foundation, emulsion foundation, cleansing cream, cleansing foam, cleansing water, soap, ointment, pack, stick products, balm type products, spray, or powder.

When the cosmetic composition according to the present disclosure has a cream or gel formulation, it may further include, as the carrier component, animal oil, vegetable oil, wax, paraffin, starch, cellulose derivatives, polyethylene glycol, silicone, bentonite, silica, talc, or zinc oxide.

When the cosmetic composition has the solution or emulsion formulation, water, ethanol, isopropanol, ethyl carbonate, ethyl acetate, benzyl alcohol, propylene glycol, glycerol aliphatic esters, polyethylene glycol, or sorbitan fatty acid esters may be added further as a solvent, a solvating agent, or an emulsifying agent.

When the cosmetic composition has the suspension formulation, liquid diluents such as water, ethanol, or propylene glycol, suspensions such as ethoxylated isostearyl alcohol, polyoxyethylene sorbitol ester, and polyoxyethylene sorbitan ester, microcrystalline cellulose, aluminum metahydroxide, bentonite, agar, or tragacanth may be included further as a component of the carrier.

If the cosmetic composition has the powder or spray formulation, lactose, talc, silica, aluminum hydroxide, calcium silicate or polyamide powder may be included further as the carrier component, and in particular, in the case of the spray formulation, boosters such as chlorofluorohydrocarbon, propane/butane, or dimethyl ether may be additionally included.

When the cosmetic composition has the surfactant-containing cleansing formulation, aliphatic alcohol sulfate, aliphatic alcohol ether sulfate, sulfosuccinic acid monoester, isethionate, imidazolinium derivative, methyltaurate, sarcosinate, fatty acid amide ether sulfate, alkylamidobetaine, aliphatic alcohol, fatty acid glycerides, fatty acid diethanolamide, vegetable oil, linolin derivatives, or ethoxylated glycerol fatty acid ester may be included further as the carrier component.

The cosmetic composition according to the present disclosure may be applied once or twice or may be applied in combination with other cosmetic compositions other than the present disclosure. The cosmetic composition according to the present disclosure may be applied once or twice or may be applied in combination with other cosmetic compositions other than the present disclosure.

### Modes for Carrying Out the Disclosure

Hereinafter, example embodiments will be described in detail to help the understanding of the present disclosure. However, the following example embodiments are merely illustrative of the content of the present disclosure, and the scope of the present disclosure is not limited to the following example embodiments. The example embodiments of the present disclosure are provided to more completely explain the present disclosure to those skilled in the art.

### <Example 1> Preparation of peptides or variants thereof

Customized by Anygen (http://www.anygen.com), peptides having amino acid sequences shown in Table 1 were prepared.

**TABLE 1**

| Name | Sequence |
|---|---|
| A900 | GCVTVLRCGEIV |
| A901 | GCETVLRCGEIV |
| A902 | GCVTELRCGEIV |
| A903 | GCETELRCGEIV |
| A904 | GCVSVLRCGEIV |
| A905 | GCVTVIRCGEIV |
| A906 | GCVTVLLCGEIV |
| A907 | GCESEII,CGEIV |
| A909 | GCVCGEIV |
| A910 | GCVTCGEIV |
| A911 | GCVTVCGEIV |
| A912 | GCVTVLCGEIV |
| A913 | GCRCGEIV |
| A914 | GCLRCGEIV |
| A915 | GCVLRCGEIV |
| A916 | GCTVLRCGEIV |

In addition, the A900 peptide variants shown in Table 2 below were prepared. Preparation was carried out via palmitoylation, hexanoylation, stearoylation, myristoylation, maleimidobutyrylation, addition of 2 polyethylene glycol (PEG2), or addition of 4 polyethylene glycol (PEG4) at the N terminus of the A900 peptides.

**TABLE 2**

| Name | Sequence (N-terminus --- C-terminus) |
|---|---|
| A900P | Palmitoyl-GCVTVLRCGEIV |
| A900H | Hexanoyl-GCVTVLRCGEIV |
| A900S | Stearoyl-GCVTVLRCGEIV |
| A900M | Myristoyl-GCVTVLRCGEIV |
| A900Mal | Maleimidobutyrol-GCVTVLRCGEIV |
| A900PEG2 | PEG2-GCVTVLRCGEIV |
| A900PEG4 | PEG4-GCVTVLRCGEIV |

### <Experimental Example 1> Identification of therapeutic effects of peptides in atopic dermatitis-induced mouse models

Experimental mice were bred in a 12-hour light/dark cycle, free of pathogens at a constant temperature of 23°C and 55% humidity. After an adaptation period of several days, DNCB-induced atopic dermatitis was induced in mice by the following method.

First, the hair in the dorsal skin area was carefully shaved using a hair removal cream, and then 100 µL of 1% dinitrochlorobenzene (DNCB) dissolved in acetone was locally treated to exposed skin once a day for 3 days to sensitize the skin and induce atopic dermatitis. It was found that atopic dermatitis was developed on day 7 by treating 120 µL of 2% DNCB for 4 to 6 days.

* 2% DNCB : Prepared by dissolving 100 mg of DNCB powder in 20 mL acetone.

* 1% DNCB : Prepared by dissolving 50 mg of DNCB powder in 20 mL acetone.

A positive control group obtained by treating mice having atopic dermatitis with anti-inflammatory moisturizing cream (Control Cream) and Dupixent injection which is a therapeutic agent for atopic dermatitis was compared with a peptide (A900 peptide) treated group.

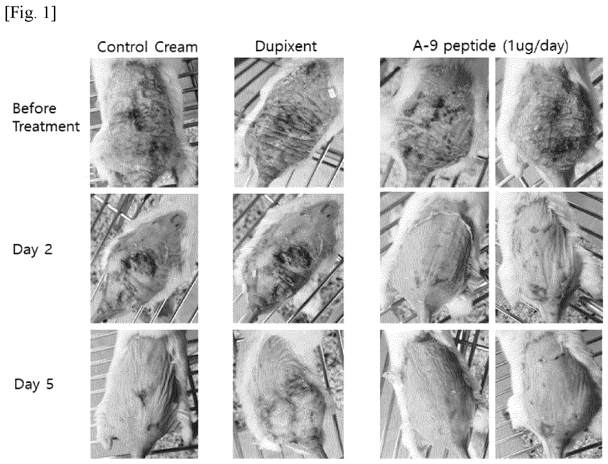

As shown in FIG. 1, when the peptide was applied using an external preparation to the mice having atopic dermatitis induced with dinitrochlorobenzene (DNCB), the atopic dermatitis symptoms were alleviated, and the effect appeared faster than the control group.

### <Experimental Example 2> Identification of an anti-inflammatory activity of peptides

In order to identify whether the peptide that has the effect of alleviating atopic dermatitis inflammation has an immunoregulatory function, splenocytes and peripheral blood leukocytes (PBLs) were separately isolated after administrating the external preparation into the mouse skin for five days, surface staining was performed with antibodies for FACS, and then the frequency of cells expressing interleukin 4 receptor (IL-4R) alpha, C-C chemokine receptor type 3 (CCR3), and cytotoxic T-lymphocyte-associated protein 4 (CTLA-4) was analyzed via flow cytometry. Dupixent was administered in a form of a peritoneal injection and tested as a control group.

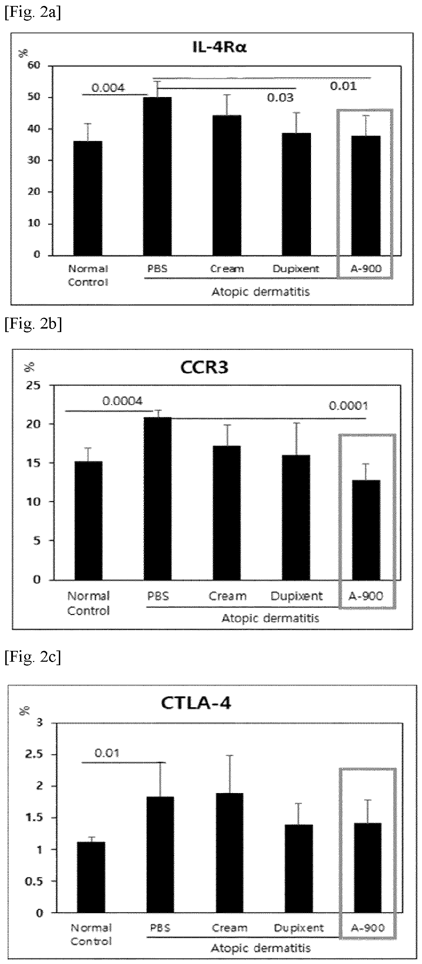

As a result of analyzing the anti-inflammatory activity of peptides using splenocytes, as shown in FIG. 2, it was observed that the frequency of IL-4R alpha, CCR3, and CTLA-4 positive cells in mice having atopic dermatitis increased compared to normal control groups, and all three molecules tended to be inhibitive after treatment of the peptide A900, while a degree of inhibition was more effective than that of the control administered group.

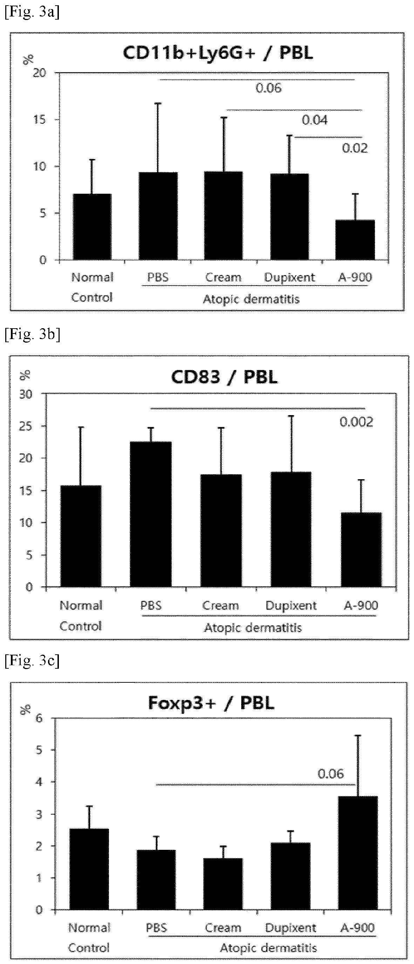

As a result of analyzing the anti-inflammatory activity of peptides using peripheral blood leukocytes (PBLs) of mice having atopic dermatitis, as shown in FIG. 3, it was observed that the frequency of CD11b+Ly6G+ and CD83+ positive cells was inhibited in a group administered with the A900 peptide while the frequency of Foxp3+ positive cells increased. It was shown that the degree of inhibition of CD11b+Ly6G+ and CD83+ positive cells was more effective than in the control administered group. This demonstrates that the relief of atopic dermatitis symptoms by administration of A900 peptide is regulated by neutrophil inhibition, CD83 inhibition, and an increase in T regulatory cells.

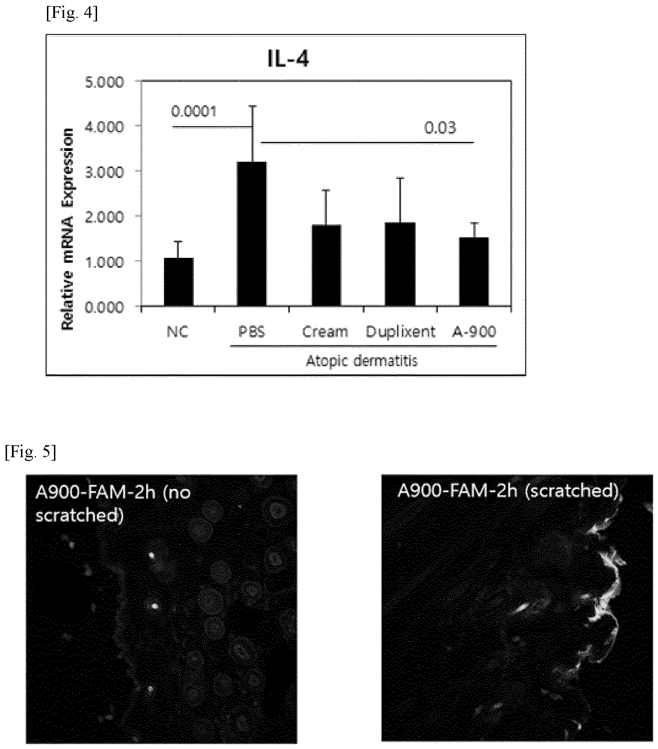

Moreover, after treating mice having atopic dermatitis with the A900 peptide, as a result of isolating cells from splenocytes and analyzing the expression level of IL-4 by real-time PCR, as shown in FIG. 4, it was shown that IL-4 expression was inhibited in the A900 peptide administered group. This demonstrates that the A900 peptide may alleviate atopic dermatitis symptoms by inhibiting IL-4 expression.

### <Experimental Example 3> Analysis of skin tissue penetrability of peptides

To determine the skin tissue penetration of the A900 peptide, peptide A900 peptide (A900-FAM) with fluorescent substance FAM (Carboxyfluorescein) attached to the N-terminus of A900 peptide was applied to the skin of ICR 4-week-old mice. As a result of making needle scratch on the skin of the right leg with no scratch on the skin of the left leg, applying 1 µg of peptide using a cotton swab, collecting skin tissues 2 hours later, and freeze-cutting for observation under a confocal microscope, as shown in FIG. 5, fluorescence was detected in both the epidermis and dermis in all the A900 peptide treated groups regardless of the presence of scratch. This demonstrates the possibility of penetration of A900 peptide through the skin tissue.

### <Experimental Example 4> Analysis of peptide expression in immune organs after fluorescently labeled peptide injection

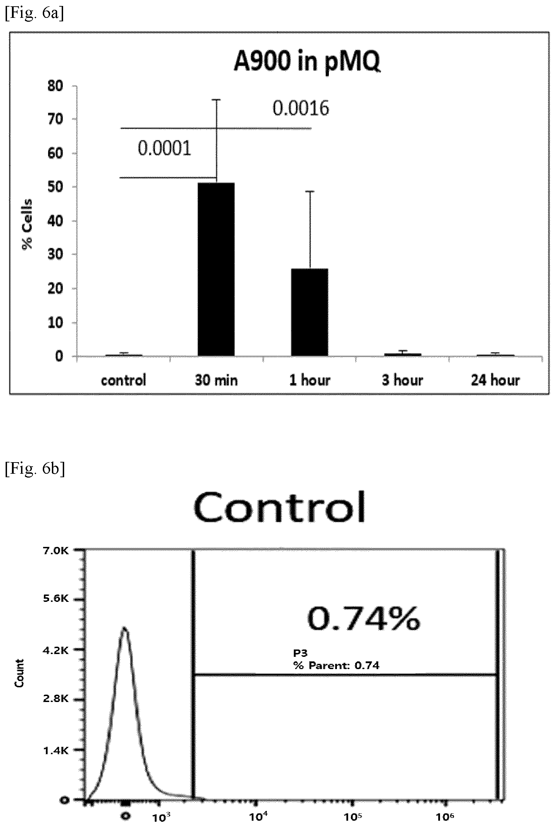

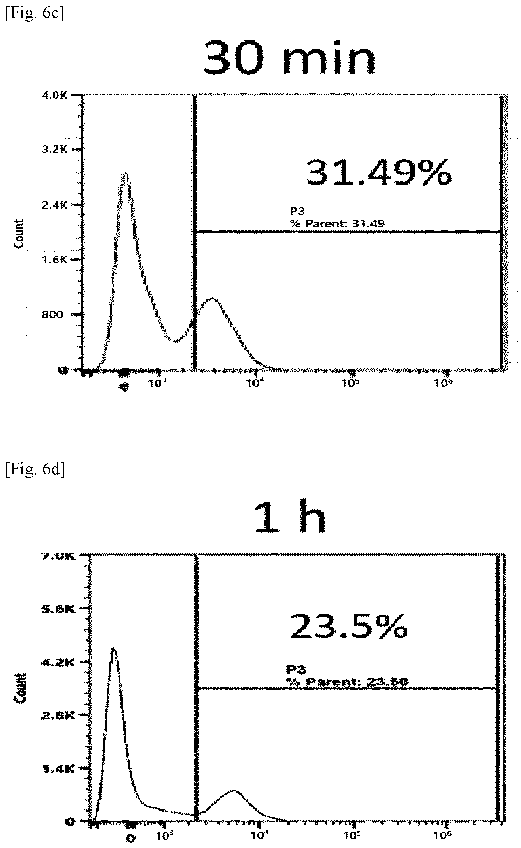

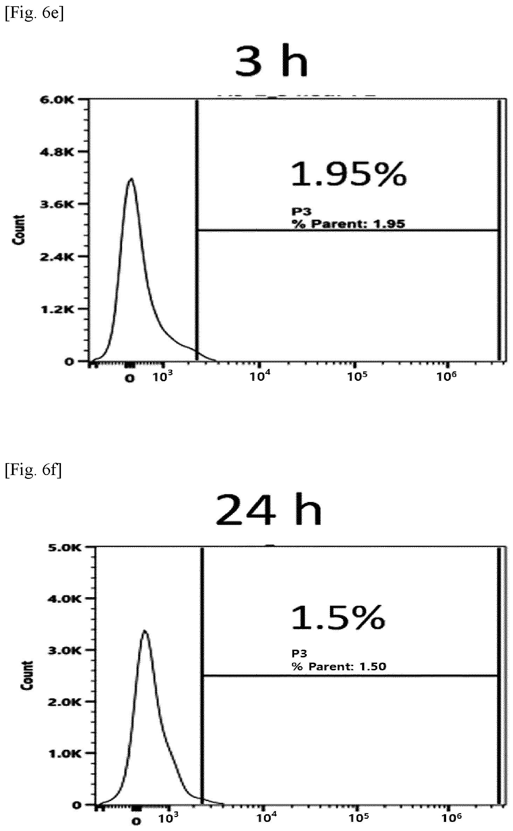

As a result of administering 3 µg of fluorescently labeled peptide to the mouse abdominal cavity and analyzing the frequency of fluorescence-expressing immune cells over time by FACS, as shown in FIG. 6, 50% of the peritoneal macrophages expressed fluorescence at 30 minutes, 23% thereof expressed fluorescence at 1 hour, and fluorescence-expressing cells were barely detected after 3 hours. This demonstrates that the peptide administered intraperitoneally passes through the membrane of the cell in the abdominal cavity and enters the cell, where it is maintained in the cell for more than 1 hour.

### <Experimental Example 5> Analysis of the intracellular expression and frequency after treating cells with peptides

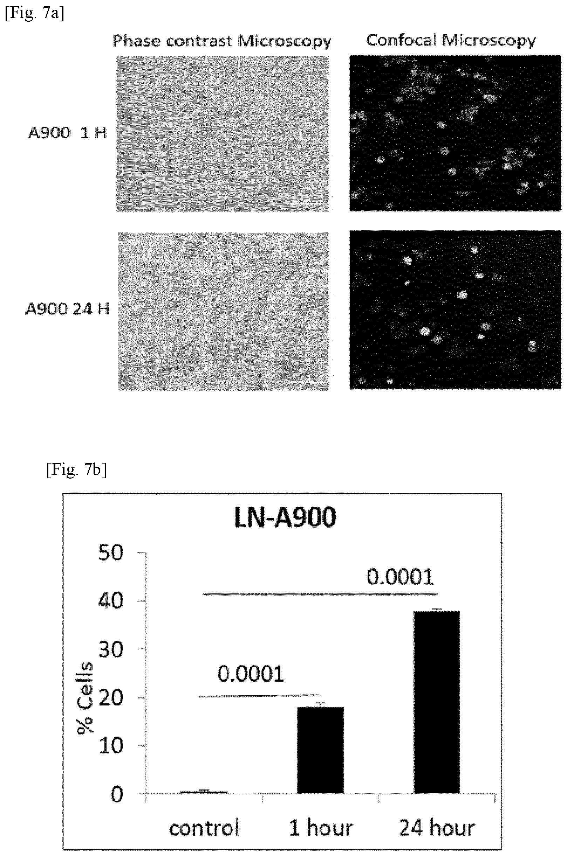

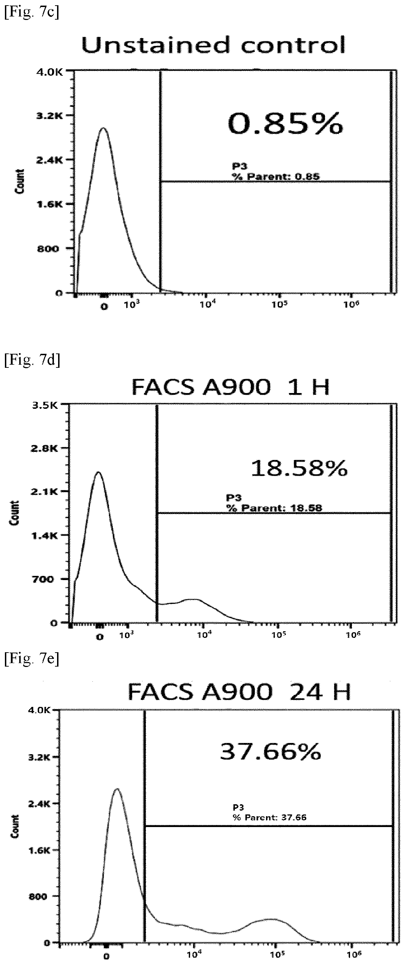

Lymph node cells were isolated from male ICR mice aged from 5 to 6 weeks, and cell suspension was cultured. Anti-CD3 (1 µg/ml) antibody was treated and coated in a petri dish at room temperature for 1 hour, and then the cells were inoculated (5 x 10⁵ cells/well). After 1 hour, anti-CD28 antibodies (1 µg/ml), IL-23 (20 ng/ml), IL-6 (20 ng/ml), and TGF-β (5 ng/ml) were added. Thereafter, as a result of treating cells cultured for 3 days with A900-FAM peptide (1 µg/mL) for 1 hour and 24 hours to analyze the intracellular expression of peptides and expression cell frequency through confocal microscopy and FACS, as shown in FIG. 7, cells with green fluorescently labeled peptide were identified, and peptides were expressed at a cell frequency of 20% in the 1-hour treated group and 40% in the 24-hour treated group, showing that the expression and frequency of peptides increased over time.

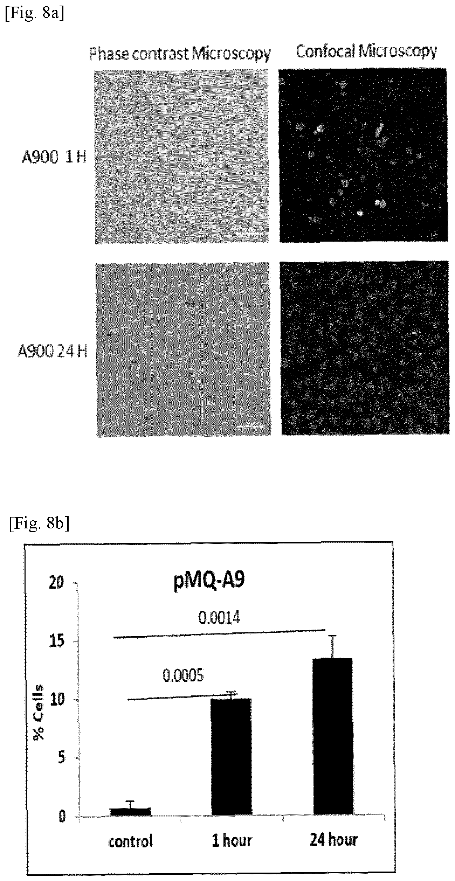

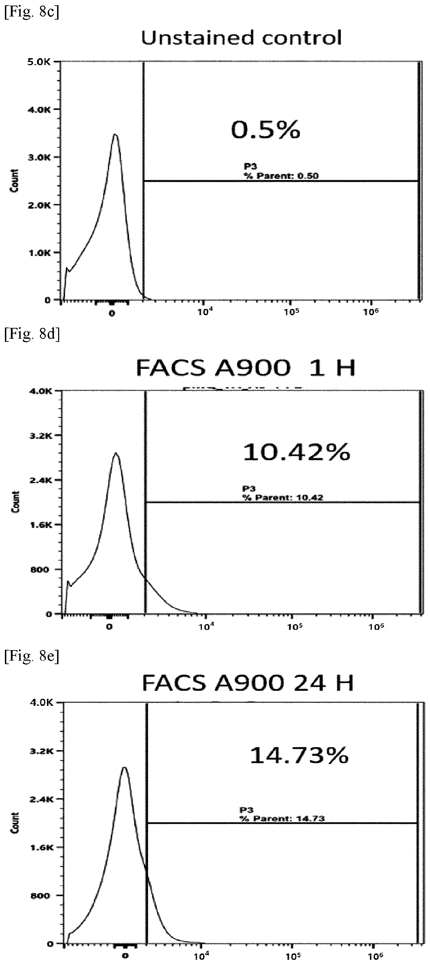

Peritoneal macrophages were isolated from the same mouse used in FIG. 7 to culture the cell suspension. Anti-CD3 (1 µg/ml) antibodies were plated on a petri dish at room temperature for 3 hours and cells were inoculated (5 x 10⁵ cells/well). After 1 hour, anti-CD28 antibodies (1 µg/ml), IL-23 (20 ng/ml), IL-6 (20 ng/ml), and TGF-β (5 ng/ml) were added. Thereafter, as a result of treating cells cultured for 3 days with A900-FAM peptide (1 µg/mL) for 1 hour and 24 hours to analyze the intracellular expression and frequency through confocal microscopy and FACS, as shown in FIG. 8, the peptides were expressed at a frequency of 10% in the 1-hour culture group and 15% in the 24-hour culture group, showing that the expression frequency of peptides increased over time.

### <Experimental Example 6> Analysis of effects of the peptide in spleen after treating mouse skin having atopic dermatitis with peptides

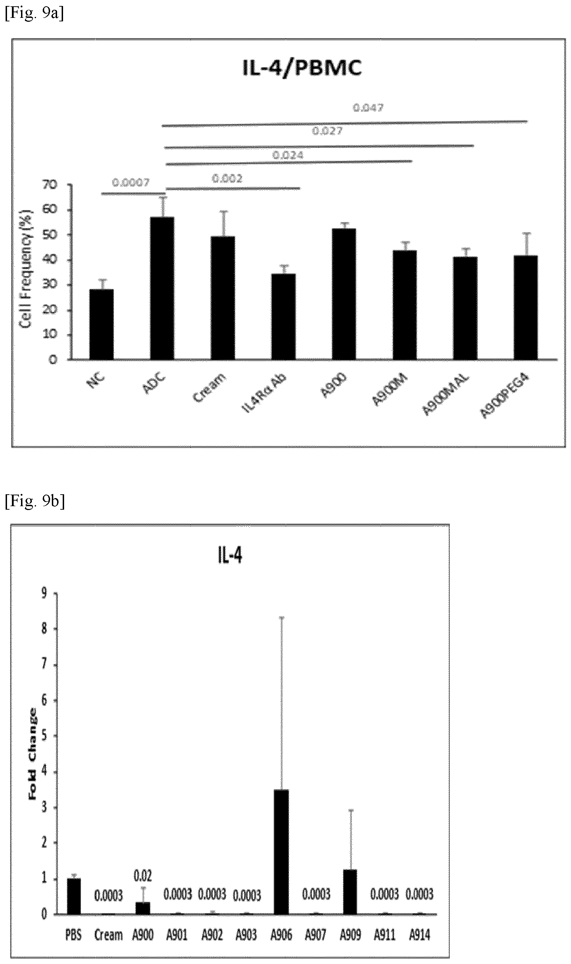

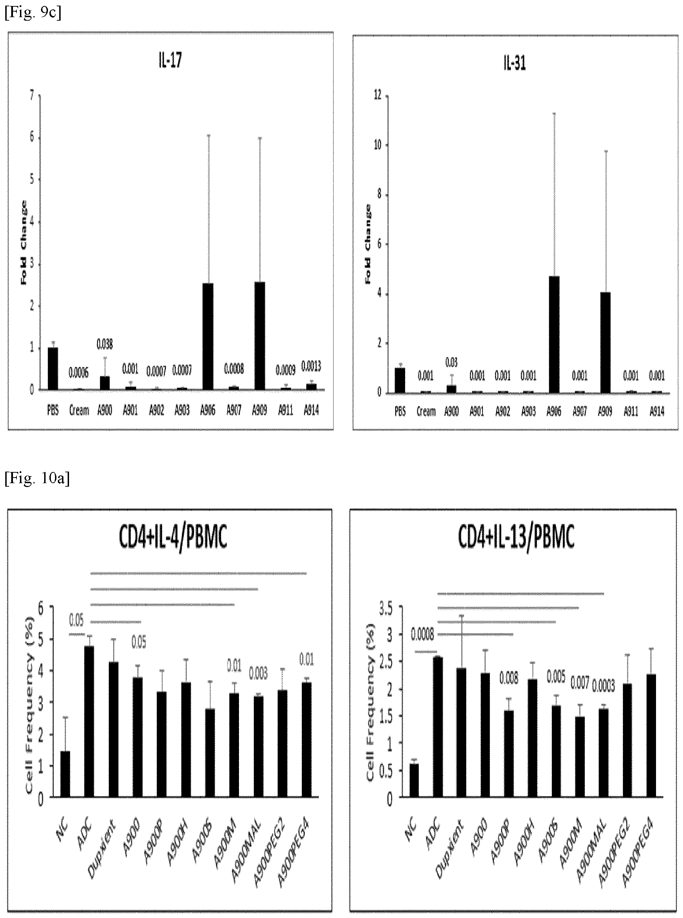

As a result of treating skin lesions of mice having atopic dermatitis with the peptide in a form of external preparation (A900~A916) and analyzing the effect of the peptide through real-time PCR in the spleen, as shown in FIG. 9, it was shown that A900, A901, A902, A903, A907, A911, and A914 peptides inhibited expression of IL-4, IL-31, and IL-17, which are atopy associated cytokines.

### <Experimental Example 7> Analysis of FACS results via ex vivo

After removing hair from 6-week-old Balb/c male mice, application was performed with 1% DNCB 1 time/day for 2 days and 0.5% DNCB 1 time/day for 3 days for a total of 5 days to induce atopic symptoms. PBMCs were isolated from atopic mice, seeded in 24 well plates, treated with peptides, cultured for 24 hours, and then analyzed by FACS. PBMCs isolated from normal mice and atopy-induced mice were used as control groups, and peptides were treated to PBMC cultures isolated from atopic mice. Dupixent was used for the peptide control group (Dupixent 5 µg/ml). A900, A900P, A900H, A900S, A900M, A900MAL, A900PEG2, and A900PEG4 were used as the peptides, and the concentration was set to 3 µg/ml. Surface was stained with anti-CD4 antibodies, cytokines were subjected to intracellular staining, and the frequency of double stained population was measured.

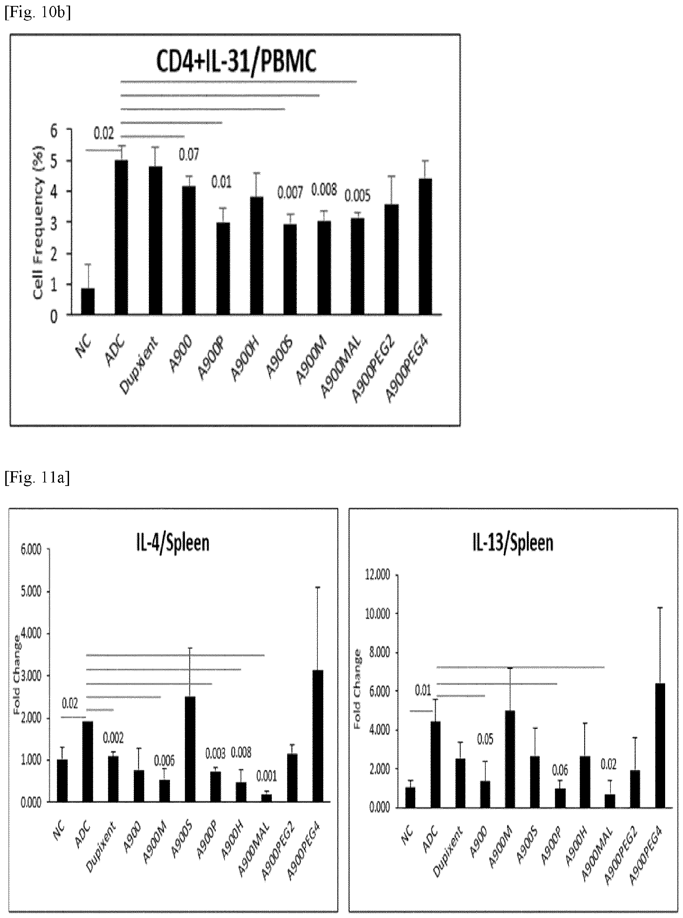

As a result, as shown in FIG. 10, compared to the untreated control group (ADC), A900, A900M, A900MAL, A900PEG4, A900P, and A900S peptides inhibited the frequency of CD4 T cells secreting atopy associated cytokines, showing immunoregulatory effects associated with atopic dermatitis symptoms.

### <Experimental Example 8> Analysis of real-time PCR results of intracellular cytokines

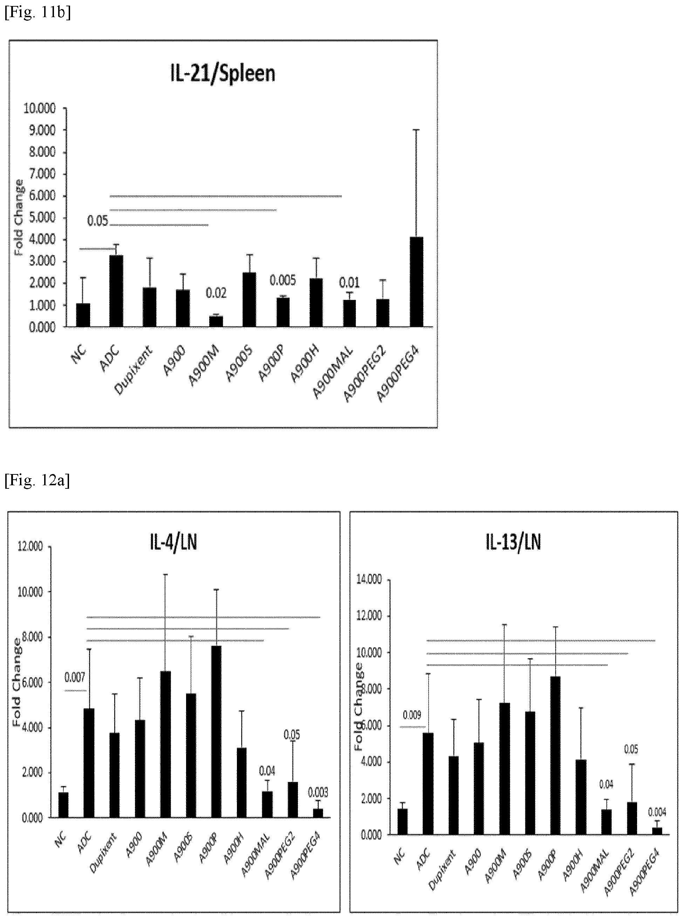

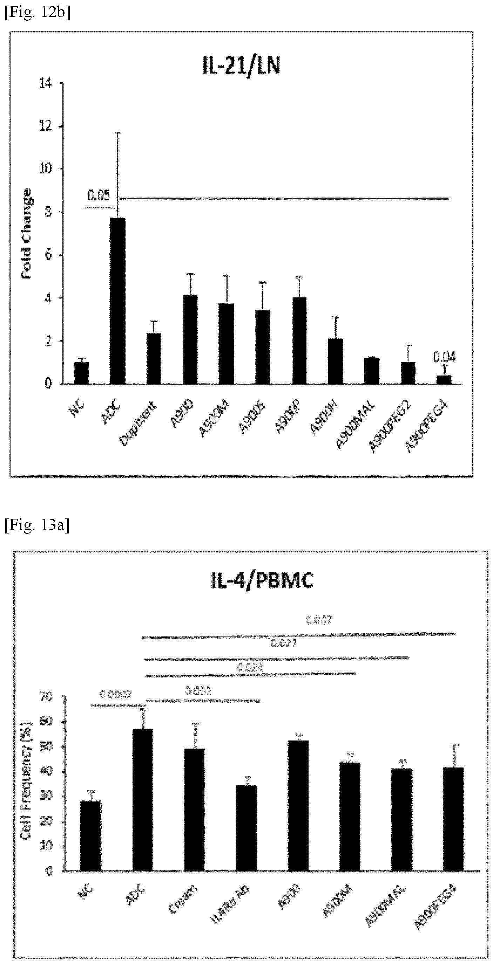

As a result of treating splenocytes and lymph node cells isolated from the same mouse as in FIG. 10 with each of A900 and A900 peptide terminal conjugated peptides (A900M, A900S, A900P, A900H, A900MAL, A900PEG2, and A900PEG4 peptides), culturing for 24 hours, and analyzing a degree of cytokine expression associated with induction of atopic dermatitis symptoms by real time PCR, as shown in FIGS. 11 and 12, it was shown that, compared to the untreated control group (ADC), A900, A900M, A900P, A900H, A900MAL, A900PEG2, and A900PEG4 peptides inhibited IL-4, IL-13, and IL-21 which are atopy associated cytokines.

### <Experimental Example 9> Analysis of FACS results of intracellular cytokines via an in vivo experiment

After removing hair from 6-week-old Balb/c male mice, application was performed with 1% DNCB 1 time/day for 2 days and 0.5% DNCB 1 time/day for 3 days for a total of 5 days, to induce atopic symptoms (N=5~8 mice for each group). After administering the external preparation to mouse skin for 5 days in mice having atopic symptoms, peripheral blood leukocytes (PBLs) were isolated and the frequency of each cytokine expressing cell was analyzed and compared after intracellular staining with antibodies for FACS. The control group was given with an anti-inflammatory moisturizing cream external preparation (5 times) and administered with an anti-mouse IL-4 receptor antibody (100 ug/mouse) intraperitoneal injection (1 time). A900, A900M, A900MAL, and A900PEG4 were used for peptides, and the concentration was set to 1 µg/ml. Cytokines were subjected to intracellular staining to measure the frequency of stained cell population.

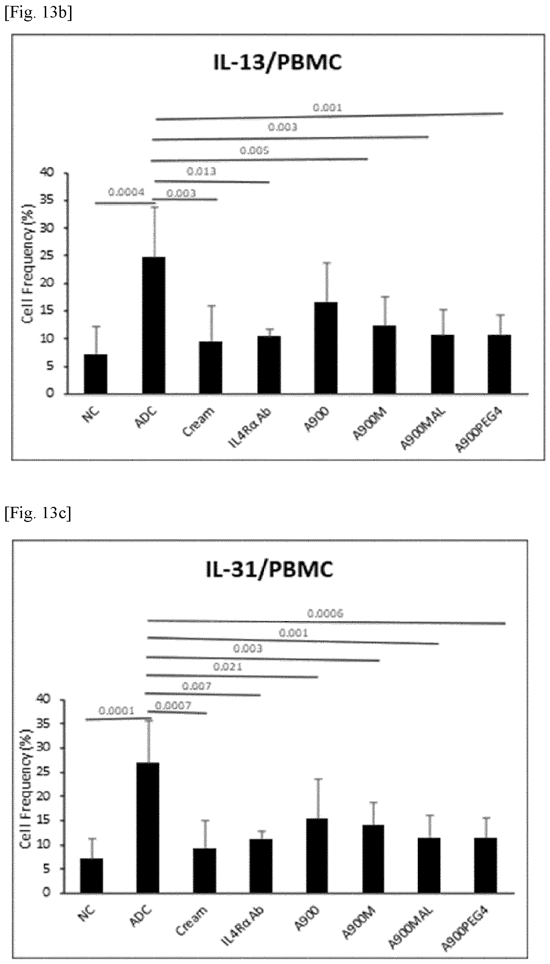

As a result, as shown in FIG. 13, compared to mice having atopic dermatitis (ADCs) administered with PBS, the control group, it was shown that A900, A900M, A900MAL, and A900PEG4 peptides inhibited the frequency of cells secreting atopy associated cytokines, showing immunoregulatory effects associated with atopic dermatitis symptoms.

Peripheral blood leukocytes (PBLs) isolated from the same mice as in FIG. 13 were isolated and subjected to CD4 surface staining and intracellular staining with antibodies for FACS to analyze and compare the frequency of each cytokine-expressing CD4 cell.

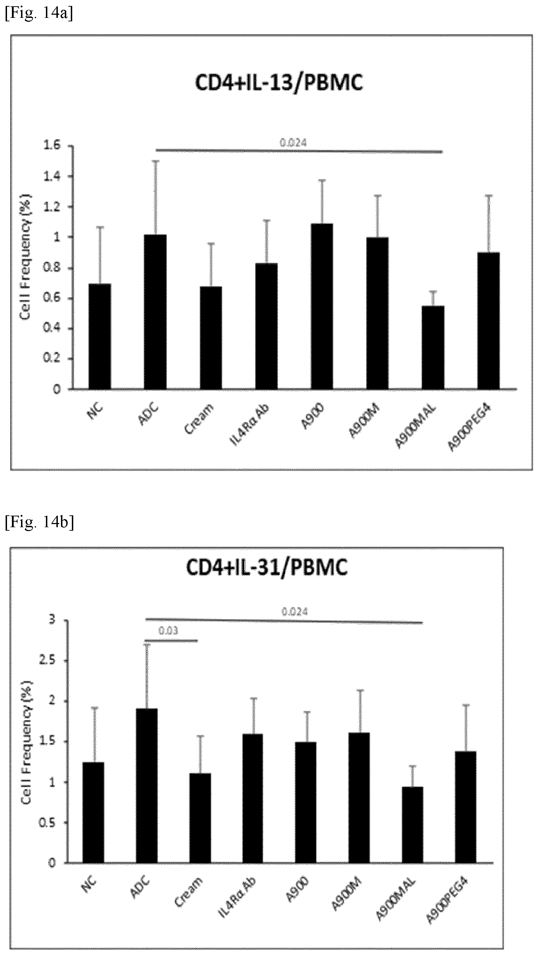

As a result, as shown in FIG. 14, it was shown that the A900MAL peptide inhibited the frequency of CD4 cells secreting atopy associated cytokine IL-13 and CD4 cells secreting IL-31, showing immunoregulatory effects associated with atopic dermatitis symptoms.

As described above, a specific part of the present disclosure has been described in detail, and it is apparent to those skilled in the art that such specific descriptions are only preferred example embodiments and the scope of the present disclose is not limited thereby. That is, the substantial scope of the present disclosure is defined by the appended claims and their equivalents.

## Claims

1. A peptide or fragment thereof, comprising an amino acid sequence selected from the group consisting of SEQ ID NO: 1 to SEQ ID NO: 17.

2. The peptide or fragment thereof of claim 1, wherein the peptide or fragment thereof has an immunoregulatory function or anti-inflammatory activity.

3. The peptide or fragment thereof of claim 1, wherein the peptide or fragment thereof penetrates skin tissues and passes through cell membranes.

4. The peptide or fragment thereof of claim 1, wherein the peptide or fragment thereof inhibits the frequency of CD4 T cells to suppress cytokines, and thus has an activity in atopic dermatitis.

5. The peptide or fragment thereof of claim 4, wherein the cytokine is one or more selected from the group consisting of IL-4, IL-13, IL-17, IL-21, and IL-31.

6. A conjugate in which the peptide or fragment thereof according to claim 1 is conjugated with a biocompatible polymer or fatty acid.

7. The conjugate of claim 6, wherein the biocompatible polymer is one or more selected from the group consisting of pullulan, chondroitin sulfate, hyauronic acid (HA), glycol chitosan, starch, chitosan, dextran, pectin, curdlan, poly-L-lysine, poly-aspartic acid (PAA), polylactic acid (PLA), polyglycolide (PGA), poly(ε-caprolactone) (PCL), poly(caprolactone-lactide) random copolymer (PCLA), poly(caprolactone-glycolide) random copolymer (PCGA), poly(lactide-glycolide) random copolymer (PLGA), polyethylene glycol (PEG), pluronic F-68, and pluronic F-127.

8. The conjugate of claim 6, wherein the fatty acid is one or more selected from the group consisting of hexanoic acid, caprylic acid, capric acid, maleimidobutyric acid, lauric acid, myristic acid, palmitic acid stearic acid, and cholesterol.

9. A pharmaceutical composition for preventing or treating atopic dermatitis, comprising, as an active ingredient, one or more selected from the group consisting of the peptide or fragment thereof according to claim 1, and the conjugate according to claim 6.

10. The pharmaceutical composition of claim 9, wherein the peptide or fragment thereof, or the conjugate is included in an amount of 0.001 to 50 parts by weight with respect to 100 parts by weight of the total composition.

11. The pharmaceutical composition of claim 9, wherein composition is selected from the group consisting of oral preparations, external preparations, suppositories, and injections.

12. A cosmetic composition for preventing or ameliorating atopic dermatitis, comprising, as an active ingredient, one or more selected from the group consisting of the peptide or fragment thereof according to claim 1, and the conjugate according to claim 6.

13. The cosmetic composition of claim 12, wherein the peptide or fragment thereof, or the conjugate is included in an amount of 0.001 to 50 parts by weight with respect to 100 parts by weight of the total composition.

14. The cosmetic composition of claim 12, wherein the composition has a formulation of softening toner, nourishing toner, nourishing cream, gel, essence, soap, ointment, balm, or pack.
